# EUROPÄISCHE PATENTSCHRIFT

(11) **EP 3 518 833 B1**
(45) Veröffentlichungstag und Bekanntmachung des Hinweises auf die Patenterteilung: **31.08.2022**
(21) Anmeldenummer: 17783420.7
(22) Anmeldetag: 27.09.2017
(51) Int. Cl.: A61F 2/36

(54) **IMPLANTAT MIT EINER BEWEGBAREN IMPLANTATKOMPONENTE**
IMPLANT HAVING A MOVABLE IMPLANT COMPONENT
IMPLANT MUNI D'UN ÉLÉMENT MOBILE

(30) Priorität: 30.09.2016 DE 102016219086
(43) Veröffentlichungstag der Anmeldung: 07.08.2019
(73) Patentinhaber: Fraunhofer-Gesellschaft zur Förderung der angewandten Forschung e.V., 80686 München (DE)
(72) Erfinder: ROTSCH, Christian, 01468 Moritzburg (DE); SENF, Björn, 01259 Dresden (DE); FLÜGEL, Felix, 01277 Dresden (DE); GÖDICKE, Christoph, 04600 Altenburg (DE); GILLE, Katarina, 01159 Dresden (DE); ETTRICHRÄTZ, Martin, 09126 Chemnitz (DE); HUNGER, Sandra, 09126 Chemnitz (DE)
(74) Vertreter: Pfenning, Meinig & Partner mbB
(86) Internationale Anmeldenummer: PCT/EP2017/074476
(87) Internationale Veröffentlichungsnummer: WO 2018/060235

(56) Entgegenhaltungen:
- EP-A1- 2 716 261
- WO-A2-2006/051547
- DE-A1-102005 045 070
- US-A- 5 415 660

## Beschreibung

Die Erfindung betrifft ein Implantat mit einer bewegbaren Implantatkomponente.

Die Anforderungen an Implantate wachsen stetig. So sollen beispielsweise Hüftimplantate nicht mehr nur 15 Jahre im Körper verbleiben können, sondern nach Möglichkeit ein ganzes Leben. Zudem werden die Patienten jünger, so dass langlebige und zum Teil mitwachsende Implantate benötigt werden, um Revisionsoperationen zu vermeiden.

Durch diese Anforderungen ist es notwendig, Implantate zu funktionalisieren und die Funktionen gezielt steuern zu können. Solche Funktionen können eine Geometrieänderung in Länge, Umfang, Volumen oder Oberfläche, eine zusätzliche Verankerung des Implantats oder eine Freigabe von Medikamentendepots sein. Eine Längen- und/oder Umfangsänderung könnte zum Beispiel für ein mitwachsendes Implantat von Bedeutung sein. Die zeitlich gesteuerte Freigabe von Medikamentendepots könnte eine Entzündung und damit verbundene nachfolgende Revisionsoperationen vermeiden.

Für die dargestellte Problematik hat sich am Markt noch keine Lösung etabliert. Allerdings bestehen bereits Teillösungen, die die gestellten Anforderungen auf verschiedenste Weise lösen.

So sind beispielsweise Hüftimplantate bekannt, die im proximalen Bereich an ihrer Oberfläche Elemente, die mit Formgedächtnismetallen gebildet sind, aufweisen. Diese Elemente verformen sich bei einer Erwärmung auf Körpertemperatur und sollen so die Stabilität im Knochen erhöhen. Das Auslösen der Elemente erfolgt demnach direkt nach dem Einbringen des Implantats in den Körper und ist zeitlich später nicht mehr beeinflussbar.

Weiterhin ist ein System zur Verlängerung des Schienbein- oder Oberschenkelknochens mit einem Verlängerungsmarknagel bekannt. Hier kann die Verlängerung des Marknagels durch den Patienten gesteuert werden. Dabei muss die Energie zur Verlängerung des Marknagels über Energiespeicher, die ebenfalls in den Körper implantiert werden, bereitgestellt werden.

So ist aus WO 2006/051547 A2 eine scheibenförmige Prothese bekannt.

Es ist also Aufgabe der Erfindung, ein Implantat mit einer beweglichen Implantatkomponente vorzuschlagen, um eine Funktionalisierung des Implantats ohne einen zusätzlichen Energiespeicher zu erreichen und dabei diese Funktionalisierung gezielt aktivieren zu können.

Die Aufgabe der Erfindung wird mit einem Implantat, das die Merkmale des Anspruchs 1 aufweist, gelöst. Vorteilhafte Ausführungsformen und Weiterbildungen sind in den untergeordneten Ansprüchen bezeichnet.

In einem erfindungsgemäßen Implantat mit einer bewegbaren Implantatkomponente weist die bewegbare Implantatkomponente ein vorgespanntes System auf, das durch einen Auslösemechanismus gehalten ist. Dabei weist der Auslösemechanismus einen Aktor auf, der mit einem Formgedächtnismetall gebildet ist.

Durch einen drahtlosen Energieeintrag werden in dem Formgedächtnismetall eine Gefügeänderung und damit das Auslösen, also die Freigabe des vorgespannten Systems erreicht, so dass die Implantatkomponente bewegbar ist.

Der Aktor, der das vorgespannte System in einem definierten Zustand hält, kann ein Draht, eine Klammer, einer Klemme, ein Stab und/oder ein Rohr sein. Er kann auch eine andere, geometrische definierte dreidimensionale Form aufweisen, die geeignet ist, das vorgespannte System zu halten.

Das vorgespante System kann eine Feder, ein Ventil, ein Blech und/oder eine Membran aufweisen. Ist das vorgespannte System beispielsweise ein Blech, kann sich dieses Blech nach der Freigabe des Auslösemechanismus verformen und an die Knochenkontur anlegen und so die Position des Implantats beispielsweise nach einer Lockerung sichern.

Das Formgedächtnismetall sollte aus einer thermischen Formgedächtnislegierung, bevorzugt einer Nickel-Titan-Legierung gebildet sein, so dass die erforderliche Gefügeänderung zur Freigabe des Auslösemechanismus in einem definierten Temperaturbereich, vorzugsweise im Bereich von 42 °C bis 70 °C, erreichbar ist. Der definierte Temperaturbereich sollte für einen Zeitraum im Bereich von 30 s bis 3 min gehalten werden, um eine vollständige Gefügeänderung und damit ein vollständiges Auslösen der bewegbaren Implantatkomponente sicherzustellen. Die Formgedächtnislegierung kann aber auch eine magnetische Formgedächtnislegierung sein.

Der drahtlose Energieeintrag kann durch ein elektrisches Feld, ein magnetisches Feld und/oder ein elektromagnetisches Feld erreicht werden.

In einer bevorzugten Ausführungsvariante kann der Auslösemechanismus in mehreren Teilschritten ausgelöst werden. Dies kann bevorzugt durch eine Erhöhung des Energieeintrags, ganz besonders bevorzugt durch eine schrittweise Erhöhung des Energieeintrags, durch mehrere nacheinander geschaltete Teilmechanismen oder durch eine konstruktive Ausgestaltung des Auslösemechanismus, insbesondere mittels Einrastmechanismen erreicht werden.

Mit einem solchen schrittweisen Auslösen und damit einer schrittweisen Bewegung der Implantatkomponente kann eine individuelle Anpassung des Implantats, beispielsweise eine schrittweise Verlängerung des Implantats oder eine Wiederverankerung des Implantats nach einer nutzungsbedingten Lockerung zu verschiedenen Zeitpunkten nach der Implantation des Implantats, erreicht werden.

Weiterhin kann es vorgesehen sein, dass der Auslösemechanismus des erfindungsgemäßen Implantats rückstellbar ausgebildet ist, so dass der Auslösemechanismus in eine Position, bevorzugt die Ausgangsposition zurück bewegt werden kann. Die Rückstellung des Auslösemechanismus kann durch einen Zweiwegeeffekt des mit einem Formgedächtnismetall gebildeten Aktors und/oder des mit einem Formgedächtnismetall gebildeten vorgespannten Systems, mindestens einem mit einem Formgedächtnismetall gebildeten weiteren Element und/oder mittels mindestens eines zusätzlichen mechanischen Elements in Form einer Zugfeder, einer Druckfeder und/oder eines Federbleches in dem Auslösemechanismus erreicht werden. Weiterhin kann die Rückstellung des Auslösemechanismus durch eine externe Kraft auf die bewegliche Implantatkomponente, die dazu mit einer geeigneten Geometrie ausgebildet ist, und die Verwendung des Einwegeeffektes erreicht werden. Eine solche Rückstellung des Auslösemechanismus hat den Vorteil, dass beispielsweise ein Implantat vor seinem Austausch in eine Ausgangsposition zurückgesetzt werden kann, so dass das Entfernen aus dem Knochen vereinfacht wird. Außerdem kann auf diese Weise auch eine Korrektur einer Wiederverankerung, einer Verlängerung eines Implantats oder eine Korrektur der Ausrichtung des Implantats erfolgen. Außerdem kann so eine thermische Behandlung und/oder Sterilisation des Implantates vor der Implantation durchgeführt werden.

Die Oberfläche der bewegbaren Implantatkomponente kann beschichtet sein, um die Reibung zwischen der Implantatkomponente und dem Knochen, in den das Implantat eingesetzt worden ist, zu erhöhen und somit eine sichere Lage des Implantats zu erreichen und sicherzustellen, dass sich die bewegbare Implantatkomponente und der umgebende Knochen nach dem Auslösen der bewegbaren Implantatkomponente gemeinsam bewegen. Eine Beschichtung sollte bevorzugt mit biokompatiblen und/oder bioaktiven Materialien, insbesondere mit Kohlenstoff, mit keramischen Werkstoffen, insbesondere Hydroxylapatit, metallischen Werkstoffen und/oder Kunststoffen erfolgen. Weiterhin kann die Beschichtung als Barriere zwischen der bewegbaren Implantatkomponente und dem Knochen Verwendung finden.

Eine Oberfläche der bewegbaren Implantatkomponente, also auch eine Oberfläche einer Beschichtung, kann auch mikrostrukturiert sein. Auch die Mikrostrukturierung dient der Erhöhung der Reibung zwischen der Implantatkomponente und dem Knochen. Außerdem können sowohl die Beschichtung, als auch die Mikrostrukturierung das Einwachsverhalten des Knochens in das Implantat zeitlich und örtlich beeinflussen.

Mit einer Dimensionierung, insbesondere der Dicke der Elemente des vorgespannten Systems kann außerdem der Anpressdruck der beweglichen Implantatkomponente gegen den Knochen beeinflusst werden.

Ein Vorteil der beschriebenen Erfindung besteht darin, dass die erforderliche mechanische Energie zum Erzielen einer zusätzlichen Funktionalität des Implantats durch das Vorspannen direkt im Implantat gespeichert ist und lediglich durch den Auslösemechanismus freigegeben werden muss. Daher kann eine kleine Komponente aus einem Formgedächtnismetall gewählt werden, die einen geringen Energieeintrag zum Auslösen benötigt.

Ein weiterer Vorteil liegt im gezielten Auslösen der Funktion durch den definierten Energieeintrag. In vorhandenen Lösungen werden Formgedächtniselemente bereits durch die Körperwärme ausgelöst und sind somit nicht zeitlich steuerbar. Andere Systeme verwenden Motoren, um eine Implantatkomponente zu bewegen. Die offenbarte erfindungsgemäße Lösung ist damit wesentlich kompakter und einfacher aufgebaut als bekannte Systeme.

Nachfolgend soll das erfindungsgemäße Implantat anhand von Beispielen näher erläutert werden. In einzelnen Beispielen realisierte Merkmale können unabhängig vom jeweiligen gezeigten und beschriebenen Beispiel miteinander kombiniert werden.

Dabei zeigen:
Figur 1 - eine erste beispielhafte Ausführungsvariante einer bewegbaren Implantatkomponente mit einer Feder als vorgespanntem System in einer vereinfachten Darstellung,
Figur 2 a, b - eine zweite beispielhafte Ausführungsvariante einer bewegbaren Implantatkomponente mit Halteelementen in einer Führung in einer vereinfachten Darstellung,
Figur 3 - eine dritte beispielhafte Ausführungsvariante einer bewegbaren Implantatkomponente mit einem Draht als Aktor in einer vereinfachten Darstellung,
Figur 4 - eine vierte beispielhafte Ausführungsvariante einer bewegbaren Implantatkomponente, ebenfalls mit einem Draht als Aktor, in einer vereinfachten Darstellung,
Figur 5 - eine fünfte beispielhafte Ausführungsvariante einer bewegbaren Implantatkomponente mit einem magnetischen Formgedächtnismetall in einer vereinfachten Darstellung,
Figur 6 a, b - eine beispielhafte Ausführungsvariante für ein schrittweises Auslösen eines vorgespannten Systems in einer Schnittdarstellung,
Figur 7 a-c - drei beispielhafte Ausführungsformen für eine Rückstellung eines Aktors in einer Schnittdarstellung,
Figur 8 - eine weitere beispielhafte Ausführungsform für eine Rückstellung eines Aktors in einer Schnittdarstellung und
Figur 9 - noch eine weitere beispielhafte Ausführungsform für eine Rückstellung eines Aktors in einer Schnittdarstellung.

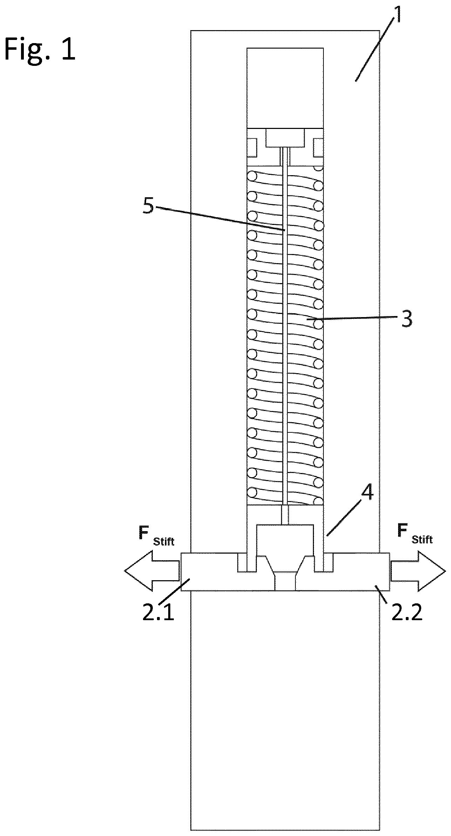

In Figur 1 ist eine erste beispielhafte Ausführungsvariante einer bewegbaren Implantatkomponente 2 dargestellt. In diesem Beispiel ist die bewegliche Implantatkomponente 2 in Form von Stiften 2.1 und 2.2 ausgebildet, die zur Verankerung eines Implantats 1 verwendet werden können. Die Stifte 2.1 und 2.2 werden von einem Auslösemechanismus 4 in einer ersten Position gehalten, in der sie ganz oder zumindest zum größten Teil innerhalb des Volumens des Implantats 1 angeordnet sind.

Der Auslösemechanismus 4 weist einen Aktor 5 in Form eines Drahtes auf, der mit einem Formgedächtnismetall, beispielsweise mit einer Nickel-Titan-Legierung gebildet ist. In diesen Draht 5 wird durch ein außerhalb des Patienten generiertes elektrisches Feld ein elektrischer Stromfluss induziert und eine Erwärmung auf eine Temperatur im Bereich von 65 °C erreicht. Dadurch wird eine Gefügeänderung erreicht, bei der Martensit in Austenit übergeht. In der Folge verkürzt sich der Draht 5, da er in eine Ausgangslänge zurück strebt. Dabei wird eine Feder zusammen gedrückt, so dass sie als vorgespanntes System 3 die Energie für eine Dehnung des Drahtes 5 in Form von Federkraft speichert.

Durch das Zusammenziehen des Drahtes 5 wird der Auslösemechanismus 4 aus den Stiften 2.1 und 2.2 herausgezogen und diese bewegen sich aufgrund der durch das vorgespannte System wirkenden Kräfte nach außen, um das Implantat 1 neu bzw. besser zu verankern. Kühlt sich der Draht 5 ab, wird der Austenit in Martensit umgewandelt. Aufgrund der Federkraft in der Feder 3 wird der Draht 5 nun gedehnt.

Werden die Stifte 2.1 und 2.2 später wieder in das Innere des Implantats 1 hineingedrückt, wird aufgrund der schrägen Kanten in den Stiften 2.1 und 2.2 der Auslösemechanismus 4 entgegen der Federkraft bewegt. Bei einem weiteren Eindrücken rasten die Stifte 2 wieder in den Auslösemechanismus 4 ein. Der Auslösemechanismus 4 befindet sich nun wieder im eingerasteten Zustand und kann dann erneut ausgelöst werden.

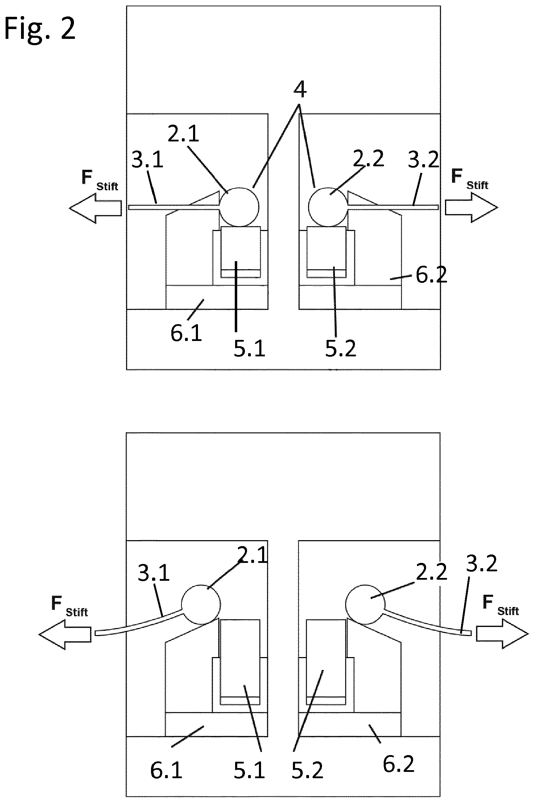

In Figur 2 wird eine zweite beispielhafte Ausführungsvariante der bewegbaren Implantatkomponente 2 gezeigt. Hier werden Halteelemente 2.1 und 2.2 eingesetzt, die jeweils aus einem biegsamen Blech 3.1 bzw. 3.2 als vorgespanntes System 3 mit einem breiteren, steifen Körper an einem im Inneren des Implantats befindlichen Ende gebildet sind. Im verankerten Zustand (Figur 2a) werden die Halteelemente 2.1 und 2.2 durch im Implantat 1 angeordnete Führungen 6.1 und 6.2 an einer nach außen gerichteten Bewegung gehindert. Die Aktoren 5.1 und 5.2, die bereits verformt wurden, werden erwärmt und streben ihre Ausgangsform an. Dabei drücken die Aktoren 5.1 und 5.2 die Halteelemente 2.1 und 2.2 aus den Führungen 6.1 und 6.2 und geben die Halteelemente 2.1 und 2.2 damit frei (Figur 2b).

Werden die Halteelemente 2.1 und 2.2 wieder nach innen gedrückt, verformen sich die Bleche 3.1 und 3.2 der Halteelemente 2.1 und 2.2 aufgrund der Schrägen, die an den Führungen 6.1 und 6.2 ausgebildet sind, immer stärker und die Biegekräfte erhöhen sich. Werden die Halteelemente 2.1 und 2.2 über die Führungen 6.1 und 6.2 in das Innere des Implantats 1 bewegt, wirken sie auf die Aktoren 5.1 und 5.2 und verformen sie. Diese Ausführungsvariante weist also auch den Vorteil der Rückstellbarkeit des Auslösemachanimus 4 auf.

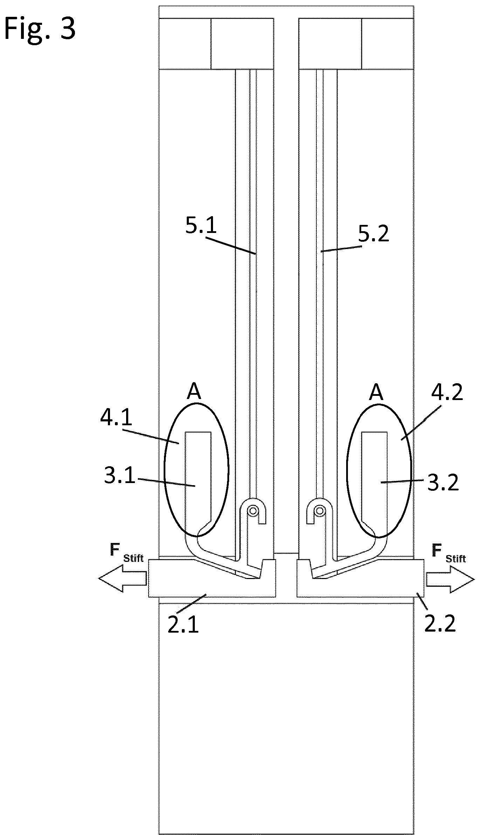

Die beispielhafte dritte Ausführungsvariante gemäß Figur 3 basiert auf dem Funktionsprinzip von Figur 1 mit Drähten 5.1 und 5.2 als Aktor 5. Das vorgespannte System 3 wird jedoch mit Rückstellelementen 3.1 und 3.2 aus einem Formgedächtnismetall im austenitischen Zustand gebildet. So kann der pseudoelastische Effekt des Formgedächntismetalls genutzt werden. Die Rückstellelemente 3.1 und 3.2 sind in dem mit "A" gekennzeichneten Bereich fest mit dem Implantat 1 verbunden. Im nicht ausgelösten Zustand dehnen die Rückstellelemente 3 die Drähte 5.1 und 5.2. Werden die Drähte 5.1 und 5.2 als Aktor 5 nun durch einen Energieeintrag erwärmt, streben diese ihren Ausgangszustand an und ziehen sich zusammen. Die Rückstellelemente 3.1 und 3.2 werden dadurch superelastisch verformt und die bewegbaren Stifte 2.1 und 2.2 freigegeben (nicht dargestellt). Diese bewegen sich aufgrund der durch das vorgespannte System erzeugten Kräfte nach außen und verankern beispielsweise das Implantat 1 im Knochen.

Kühlen die Drähte 5.1 und 5.2 ab, werden sie durch die Rückstellelemente 3.1 und 3.2 gedehnt. Werden die Stifte 2.1 und 2.2 eingedrückt, verformen sich die Rückstellelemente 3.1 und 3.2, rasten bei einer ausreichenden Verformung in die Stifte 2.1 und 2.2 ein und halten diese in der innenliegenden Position innerhalb des Implantats 1. Die Drähte 5.1 und 5.2 als Aktor 5 können nun erneut aktiviert werden.

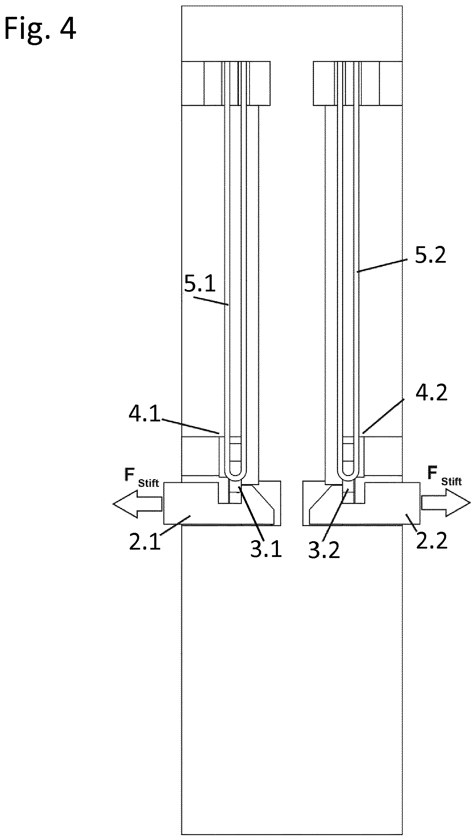

Die in Figur 4 dargestellte vierte Ausführungsvariante weist ein ähnliches Funktionsprinzip wie die dritte Ausführungsvariante auf. Die Rückstellelemente 3.1 und 3.2 sind hier allerdings mit ihrer flachen Seite senkrecht zur Kraftrichtung der Stifte 2.1 und 2.2 angeordnet und nicht fest mit dem Implantat 1 verbunden. Wie in der Ausführungsvariante nach Figur 3 sind die Rückstellelemente 3.1 und 3.2 so angeordnet, dass sie jeweils einen als Draht 5.1 und 5.2 aus einem Formgedächtnismetall ausgebildeten Aktor 5 dehnen. Werden die Drähte 5.1 und 5.2 durch einen Energieeintrag aktiviert, streben sie ihren Ausgangszustand an und ziehen sich zusammen. Dabei ziehen sie die Rückstellelemente 3.1 und 3.2 aus den Auslösemechanismen 4.1 und 4.2 und geben die Stifte 2.1 und 2.2 frei.

Im ausgelösten Zustand (nicht dargestellt) lassen sich die Stifte 2.1 und 2.2 aufgrund der schrägen Kante an ihrer Unterseite eindrücken und werden dann von den Rückstellelementen 3.1 und 3.2 gehalten. Werden die Drähte erneut durch einen Energieeintrag aktiviert, streben sie ihre Ausgangsform an und verformen sich weiter superelastisch. Die Stifte 2.1 und 2.2 werden frei gegeben.

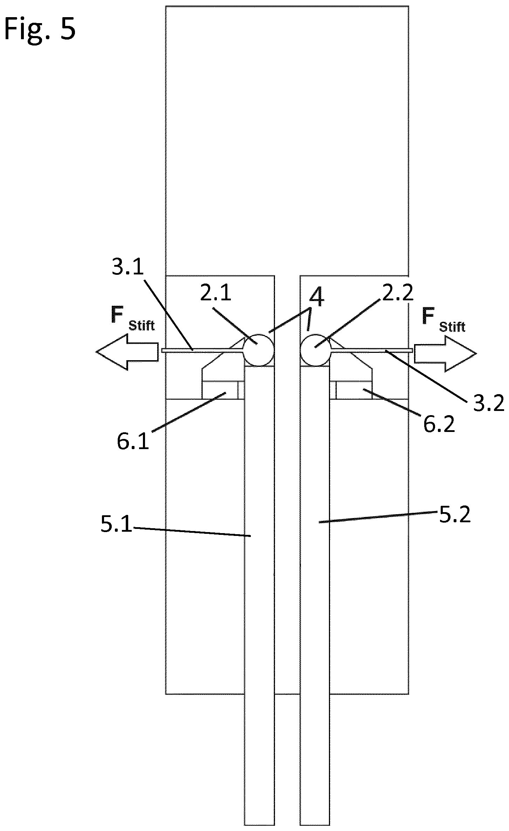

In der in Figur 5 dargestellten fünften beispielhaften Ausführungsform sind die Aktoren 5.1 und 5.2 aus einem magnetischen Formgedächtnismetall gebildet. Wie in der in Figur 2 gezeigten Ausführungsvariante werden hier Halteelemente 2.1 und 2.2 eingesetzt, die aus einem biegsamen Blech 3.1 bzw. 3.2 als vorgespanntes System 3 und mit einem breiteren, steifen Körper an einem im Inneren des Implantats 1 befindlichen Ende gebildet sind. Werden die die Bleche 3.1 und 3.2 der Halteelemente 2.1 und 2.2 beim Eindrücken gebogen, wird eine Druckkraft auf die Aktoren 5.1 und 5.2 ausgeübt. Dieser wird dadurch gestaucht und die Halteelemente 3 haken sich in der Führung 6 ein.

Wird nun ein magnetisches Feld senkrecht zur Kraftrichtung angelegt, strebt der Aktor 5 in seine Ausgangsform zurück und übt Druck auf die Halteelemente 3 aus. Diese werden dadurch über die Führung 6 hinaus bewegt und die Kräfte in den Blechen der Halteelemente 3 freigegeben.

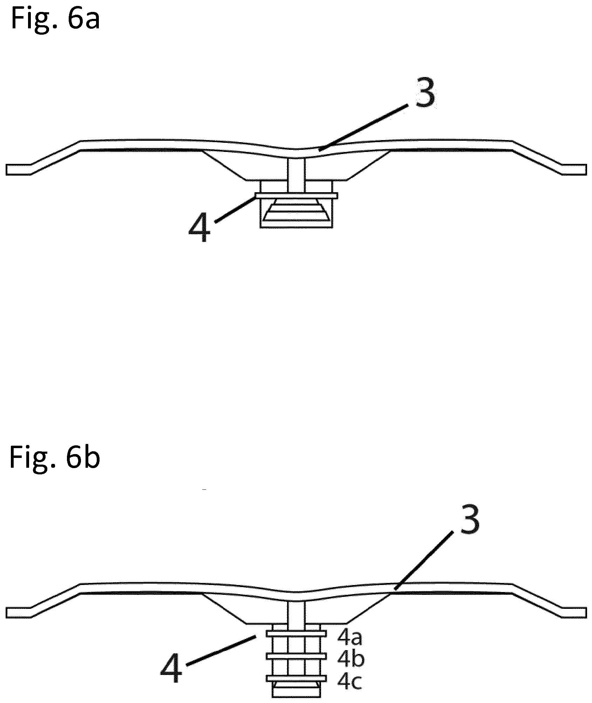

Figur 6 zeigt beispielhaft einen Auslösemechanismus 4, der schrittweise ausgelöst werden kann. Erreicht wird das schrittweise Auslösen durch drei Teilmechanismen 4a, 4b, 4c. Der aus den drei Teilmechanismen 4a, 4b, 4c gebildete Auslösemechanismus 4 hält ein vorgespanntes Blech 3 als vorgespanntes System in einer Ausgangsposition. Durch einen Energieeintrag wird zunächst der Teilmechanismus 4a ausgelöst und das vorgespannte Blech 3 kann sich in einer erste freigegebene Position bewegen und so beispielsweise an den Knochen, in den das Implantat 1 eingesetzt ist, anlegen, um eine Position des Implantats 1 zu sichern. Wenn sich das Implantat 1 nach einiger Zeit wieder lockert, kann durch einen erneuten Energieeintrag der zweite Teilmechanismus 4b und zu einem dritten Zeitpunkt der dritte Teilmechanismus 4c ausgelöst werden und so jeweils eine zweite bzw. dritte freigegebene Position durch das Blech 3 erreicht werden.

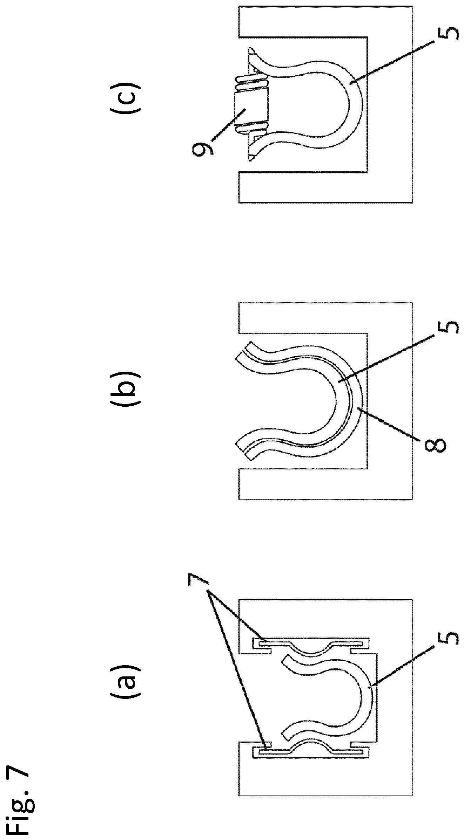

Drei beispielhafte Ausführungsformen für eine Rückstellung eines Aktors 5 sind in Figur 7 gezeigt. Der Aktor 5 umschließt dabei die bewegte Implantatkomponente 2 (nicht dargestellt) vollständig oder zumindest teilweise. Die bewegliche Implantatkomponente 2 ist in dieser beispielhaften Ausführungsvariante in Form eines Bolzens oder keilförmigen Stiftes ausgeführt und wird durch den Aktor 5 zurückgehalten bzw. vorgespannt.

Der Aktor 5 kann im in Figur 7a gezeigten Beispiel einer ersten Ausführungsvariante durch zusätzliche mechanische Komponenten in Form von zusätzlichen Federblechen 7 in eine Ausgangsposition zurückgestellt werden.

Eine weitere Ausführungsvariante ist die in Figur 7b gezeigte Lösung mit einem zusätzlichen Element 8 aus einem Formgedächtnismetall zur Rückstellung des Aktors 5. Dabei können die pseudoelastischen und/oder die pseudoplastischen Eigenschaften des zusätzlichen Elementes 8 genutzt werden. Wenn sich der Aktor nach einem Energieeintrag verformt, wird das zusätzliche Element, das form- und/oder kraftschlüssig mit dem Aktor 5 verbunden ist, mit verformt. Erfolgt im ausgelösten Zustand ein Energieeintrag, der in dem zusätzlichen Element 8 eine Gefügeänderung hervorruft, strebt dieses seine Ausgangsform an und verformt sich. Dabei wird der Aktor 5 in seine Ausgangsposition zurückgestellt.

In Figur 7c ist eine dritte Ausführungsvariante für einen Rückstellmechanismus dargestellt. Die Rückstellung eines Aktors 5 wird in diesem Fall durch eine Feder 9 erreicht. Je nach Freigaberichtung des Aktors 5 kann die Feder 9 eine Zugfeder oder eine Druckfeder sein, die den Aktor 5 in seine Ausgangsposition zurückstellt.

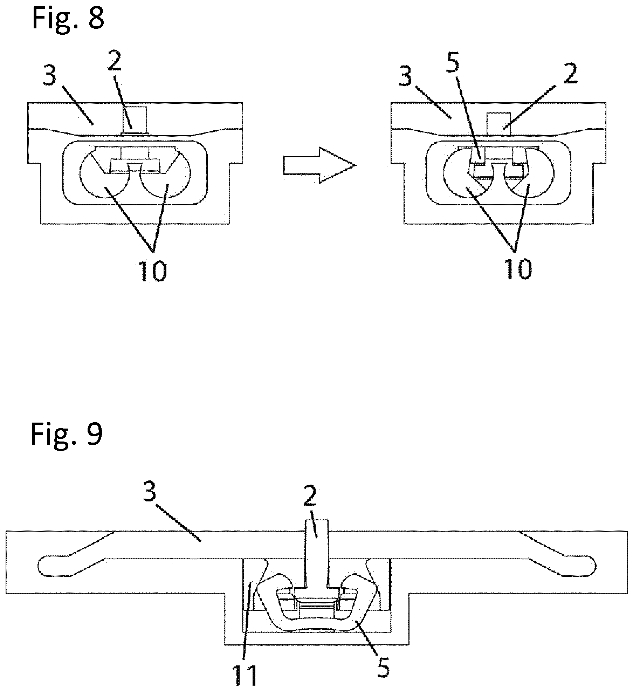

Figur 8 zeigt eine weitere beispielhafte Ausführungsform für eine Rückstellung eines Aktors 5 in Form einer Omegaklammer. Die bewegbare Implantatkomponente 2 ist in dieser Ausführungsform ein Stift, der mit einem Blech als vorgespanntes System 3 verbunden ist. Beim Eindrücken des Stiftes 2 drückt dieser auf die zylinderförmigen Rückstellelemente 10, die drehbar gelagert sind und eine Aussparung in Form eines Kreisausschnitts aufweisen. Durch den Druck des Stiftes 2 auf die Rückstellelemente 10 drehen sich diese um ihre Längsachse. Dabei stoßen sie gegen die Omegaklammer 5 als Aktor und es wirkt eine Kraft, die zu einer Verformung der Omegaklammer 5 führt. Dadurch verengt sich die Öffnung der Omegaklammer 5 so weit, dass der Stift 2 bei einer Entlastung des Bleches 3 festgehalten wird. Anschließend kann die Omegaklammer 5 durch einen Energieeintrag erwärmt und so geöffnet, und damit das Blech 3 freigegeben werden.

Figur 9 zeigt eine weitere beispielhafte Ausführungsform für eine Rückstellung eines Aktors 5. Der Aktor 5 ist in dieser Ausführungsvariante als Klammer, die aus einem Formgedächtnismetall gebildet ist, ausgebildet. Wie in Figur 8 ist die bewegbare Implantatkomponente 2 als Stift ausgebildet und fest mit einem Blech als vorgespanntes System 3 verbunden. Beim Eindrücken des Blechs 3 wird der Aktor 5 durch die Rückstellelemente 11 verformt. Die Rückstellelemente 11 weisen dazu konische Aussparungen auf. Wird der Stift 2 bzw. das Blech 3 eingedrückt, wird damit auch das Rückstellelement 11 eingedrückt. Das Rückstellelement 11 drückt damit auf die Klammer 5. Bei einem ausreichend starken Druck wird die Klammer 5 so weit verformt, dass der Stift 2 in die Klammer 5 einrasten kann. Wird die Klammer 5 durch einen Energieeintrag erwärmt, strebt sie ihren Ausgangszustand, eine weiter geöffnete Form, an und gibt damit den Stift 2 und das Blech 3 frei.

## Patentansprüche

1. Implantat (1) mit einer bewegbaren Implantatkomponente (2) für eine Funktionalisierung des Implantats (1), um Funktionen, wie eine Geometrieänderung in Länge, Volumen oder Oberfläche, eine zusätzliche Verankerung des Implantats (1) oder eine Freigabe von Medikamentendepots gezielt zu steuern,
**dadurch gekennzeichnet, dass**
die bewegbare Implantatkomponente (2) ein vorgespanntes System (3) aufweist, das durch einen Auslösemechanismus (4) gehalten ist, wobei
der Auslösemechanismus (4) einen Aktor (5) aufweist, der mit einem Formgedächtnismetall gebildet ist und
durch einen drahtlosen Energieeintrag eine Gefügeänderung in dem Formgedächtnismetall und damit das Auslösen, also die Freigabe des vorgespannten Systems (3), erreichbar ist, so dass die Implantatkomponente (2) bewegbar ist.

2. Implantat (1) nach Anspruch 1, **dadurch gekennzeichnet, dass** der Aktor (5) ein Draht, eine Klammer, eine Klemme, ein Stab und/oder ein Rohr ist.

3. Implantat (1) nach Anspruch 1 oder 2, **dadurch gekennzeichnet, dass** das vorgespannte System (3) eine Feder, ein Ventil, ein Blech und/oder eine Membran aufweist.

4. Implantat (1) nach einem der vorgehenden Ansprüche, **dadurch gekennzeichnet, dass** das Formgedächtnismetall aus einer thermischen Formgedächtnislegierung, bevorzugt aus einer Nickel-Titan-Legierung, und/oder einer magnetischen Formgedächtnislegierung gebildet ist.

5. Implantat (1) nach einem der vorgehenden Ansprüche, **dadurch gekennzeichnet, dass** der Energieeintrag durch ein elektrisches Feld, ein magnetisches Feld und/oder ein elektromagnetisches Feld erreichbar ist.

6. Implantat (1) nach einem der vorgehenden Ansprüche, **dadurch gekennzeichnet, dass** der Auslösemechanismus (4) bei einer Temperatur im Bereich von 42 °C bis maximal 70 °C auslösbar ist.

7. Implantat (1) nach einem der vorgehenden Ansprüche, **dadurch gekennzeichnet, dass** der Auslösemechanismus (4) in mehreren Teilschritten auslösbar ist, was bevorzugt durch eine bevorzugt schrittweise Erhöhung des Energieeintrags, durch mehrere nacheinander geschaltete Teilmechanismen (4a, 4b, 4c) oder durch eine konstruktive Ausgestaltung des Auslösemechanismus (4), insbesondere Einrastmechanismen erreichbar ist.

8. Implantat (1) nach einem der vorgehenden Ansprüche, **dadurch gekennzeichnet, dass** der Auslösemechanismus (4) in eine Ausgangsposition rückstellbar ist.

9. Implantat (1) nach Anspruch 8, **dadurch gekennzeichnet, dass** die Rückstellung des Auslösemechanismus (4) durch einen Zweiwegeeffekt des mit einem Formgedächtnismetall gebildeten Aktors (5) und/oder des mit einem Formgedächtnismetall gebildeten vorgespannten Systems (3), mindestens einem mit einem Formgedächtnismetall gebildeten weiteren Element und/oder mittels mindestens eines zusätzlichen mechanischen Elements in Form einer Zugfeder, einer Druckfeder und/oder eines Federbleches in dem Auslösemechanismus (4) erreichbar ist.

10. Implantat (1) nach einem der vorgehenden Ansprüche, **dadurch gekennzeichnet, dass** die äußere Oberfläche der bewegbaren Implantatkomponente (2) beschichtet ist, bevorzugt mit biokompatiblen und/oder bioaktiven Materialien, insbesondere Kohlenstoff, keramischen Werkstoffen, insbesondere Hydroxylapatit, metallischen Werkstoffen und/oder Kunststoffen beschichtet ist, und/oder mikrostrukturiert ist.

## Claims

1. An implant (1) having a movable implant component (2) for a functionalization of the implant (1), in order to control functions in a targeted manner such as a change in geometry in length, volume or surface, an additional anchoring of the implant (1) or a release of drug depots,
**characterized in that**
the movable implant component (2) comprises a prestressed system (3) held by a triggering mechanism (4), wherein
the triggering mechanism (4) comprises an actuator (5) formed with a shape-memory metal and
a structural change in the shape-memory metal and thus the triggering, that is, the release of the prestressed system (3), can be achieved by a wireless energy input, so that the implant component (2) can be moved.

2. The implant (1) according to claim 1, **characterized in that** the actuator (5) is a wire, a clip, a clamp, a rod and/or a tube.

3. The implant (1) according to claim 1 or 2, **characterized in that** the prestressed system (3) comprises a spring, a valve, a sheet and/or a membrane.

4. The implant (1) according to any one of the preceding claims, **characterized in that** the shape-memory metal is formed from a thermal shape-memory alloy, preferably from a nickel-titanium alloy, and/or a magnetic shape-memory alloy.

5. The implant (1) according to any one of the preceding claims, **characterized in that** the energy input can be achieved by an electric field, a magnetic field and/or an electromagnetic field.

6. The implant (1) according to any one of the preceding claims, **characterized in that** the triggering mechanism (4) can be triggered at a temperature in the range from 42°C to a maximum of 70°C.

7. The implant (1) according to any one of the preceding claims, **characterized in that** the triggering mechanism (4) can be triggered in a plurality of sub-steps, which is preferably achieved by a preferably gradual increase in the energy input, by a plurality of sub-mechanisms (4a, 4b, 4c) connected one after the other, or by a structural design of the triggering mechanism (4), in particular latching mechanisms.

8. The implant (1) according to any one of the preceding claims, **characterized in that** the triggering mechanism (4) can be reset to an initial position.

9. The implant (1) according to claim 8, **characterized in that** the resetting of the triggering mechanism (4) can be achieved by a two-way effect of the actuator (5) formed with a shape-memory metal and/or the prestressed system (3) formed with a shape-memory metal, at least one further element formed with a shape-memory metal and/or by means of at least one additional mechanical element in the form of a tension spring, a compression spring and/or a spring plate in the triggering mechanism (4).

10. The implant (1) according to any one of the preceding claims, **characterized in that** the outer surface of the movable implant component (2) is coated, preferably is coated with biocompatible
and/or bioactive materials, in particular carbon, ceramic materials, in particular hydroxyapatite, metallic materials and/or plastics and/or is microstructured.

## Revendications

1. Implant(1) avec un composant d'implant (2) mobile permettant une fonctionnalisation de l'implant (1), afin de commander de manière ciblée une modification de la géométrie, comme la longueur, le volume ou la surface, un ancrage complémentaire de l'implant (1) ou une libération de médicaments sous forme de dépôts,
**caractérisé en ce que**
le composant d'implant (2) mobile présente un système précontraint (3) qui est maintenu par un mécanisme de libération (4),
le mécanisme de libération (4) présentant un actionneur (5) qui est formé avec un métal à mémoire de forme, et
que par un apport d'énergie sans fil, on peut atteindre une modification de structure dans le métal à mémoire de forme et ainsi le déclenchement, c'est à dire la libération, du système précontraint (3), de sorte que le composant d'implant (2) est mobile.

2. Implant (1) selon la revendication 1, **caractérisé en ce que** l'actionneur (5) est un fil, une agrafe, une pince, une tige et/ou un tube.

3. Implant (1) selon la revendication 1 ou la revendication 2, **caractérisé en ce que** le système précontraint (3) présente un ressort, une soupape, une tôle et/ou une membrane.

4. Implant (1) selon l'une des revendications précédentes, **caractérisé en ce que** le métal à mémoire de forme est formé à partir d'un alliage à mémoire de forme thermique, de préférence, à partir d'un alliage de nickel et de titane, et/ou d'un alliage à mémoire de forme magnétique.

5. Implant (1) selon l'une des revendications précédentes, **caractérisé en ce que** l'apport d'énergie peut être réalisé par un champ électrique, un champ magnétique et/ou un champ électromagnétique.

6. Implant (1) selon l'une des revendications précédentes, **caractérisé en ce que** le mécanisme de libération (4) peut être libéré à une température dans la plage de 42 °C jusqu'à 70 °C au maximum.

7. Implant (1) selon l'une des revendications précédentes, **caractérisé en ce que** le mécanisme de libération (4) peut être déclenché en plusieurs étapes partielles, ce qui est de préférence possible par une augmentation, de préférence étape par étape, de l'apport d'énergie, par plusieurs mécanismes partiels (4a, 4b, 4c) déclenchés les uns derrière les autres ou par un aménagement de l'organisation du mécanisme de libération (4), notamment un mécanisme de verrouillage.

8. Implant (1) selon l'une des revendications précédentes, **caractérisé en ce que** le mécanisme de libération (4) peut être réinitialisé dans une position de départ.

9. Implant (1) selon la revendication 8, **caractérisé en ce que** la réinitialisation du mécanisme de libération (4) peut être réalisée par un effet bidirectionnel de l'actionneur (5) formé avec le métal à mémoire de forme, et/ou avec le système précontraint (3) formé avec un métal à mémoire de forme, avec au moins un autre élément formé avec un métal à mémoire de forme, et/ou au moyen d'au moins un élément mécanique complémentaire sous la forme d'un ressort de rappel, d'un ressort à pression, et/ou d'une tôle à ressort dans le mécanisme de libération (4).

10. Implant (1) selon l'une des revendications précédentes, **caractérisé en ce que** la surface extérieure du composant d'implant (2) mobile est revêtue, de préférence, avec des matériaux biocompatibles
et/ou bioactifs, en particulier, du carbone, des matériaux céramiques, notamment de l'hydroxyapatite, de matériaux métalliques et/ou de matières synthétiques, et/ou est microstructurée.
